# FASCICULE DE BREVET EUROPEEN

(11) **EP 1 065 982 B1**
(45) Date de publication et mention de la délivrance du brevet: **02.05.2007**
(21) Numéro de dépôt: 99909128.3
(22) Date de dépôt: 30.03.1999
(51) Int. Cl.: A61B 18/14

(54) **SONDE POUR LE TRAITEMENT HAUTE FREQUENCE DE LA PEAU**
HOCHFREQUENZ-HAUTBEHANDLUNGSSONDE
PROBE FOR SKIN HIGH FREQUENCY TREATMENT

(30) Priorité: 30.03.1998 EP 98810275
(43) Date de publication de la demande: 10.01.2001
(73) Titulaire: Bernaz, Gabriel, CH-1227 Carouge (CH)
(72) Inventeur: Bernaz, Gabriel, CH-1227 Carouge (CH)
(74) Mandataire: Cronin, Brian Harold John
(86) Numéro de dépôt international: PCT/IB1999/000553
(87) Numéro de publication internationale: WO 1999/049800

(56) Documents cités:
- EP-A- 0 129 607
- WO-A-89/00027
- FR-A- 2 589 067
- FR-A- 2 680 965
- GB-A- 2 123 287
- BIOLOGICAL ABSTRACTS, vol. 26, Philadelphia, PA, US, abstract no.: 4, LEENEN; SMITH; UNGER: "Topical minoxidil cardiac effects in bald man" page 481-486

## Description

### DOMAINE TECHNIQUE

L'invention se rapporte au traitement de la peau par des moyens électriques à haute fréquence associés ou non à une source de rayonnement électromagnétique laser, en particulier pour l'épilation dite "définitive" ou "longue durée" ainsi que pour la repousse des cheveux.

### ETAT DE LA TECHNIQUE

WO 93/04636 décrit un procédé basé sur la constatation qu'en mélangeant un gel conducteur de type usuel pour l'application de sondes à ultrasons contre la peau avec un produit traitant, par exemple une lotion à effet d'atrophie sur les racines des poils, il était possible, par induction transcutanée à haute fréquence, de faire pénétrer le produit traitant dans les follicules pileux (pores) et la tige des poils, et ainsi d'effectuer un traitement.

Ce procédé permet ainsi d'effectuer un traitement, notamment cosmétique, de la peau, par exemple en vue d'une épilation longue durée, voire définitive, et permet de surcroît une application ponctuelle et efficace au niveau des follicules, sans intervention manuelle délicate.

Pour la mise en oeuvre du procédé, WO 93/04636 décrit en outre un appareil comprenant un organe maniable de contact avec la peau ayant un corps non-conducteur pourvu d'une surface d'appui destinée à être appliquée sur la peau. Cette surface comporte une pluralité de points conducteurs d'émission électromagnétique distincts formés par exemple par des parties exposées de spires d'un solénoïde noyé, dans le corps de l'organe de contact. Ces points débouchent par des orifices dans cette surface, de préférence en retrait par rapport à celle-ci, de manière que, en cours d'utilisation de l'appareil, ils puissent contacter du gel conducteur appliqué sur la peau. Ces points d'émission distincts émettent un flux d'énergie électromagnétique à haute fréquence, avantageusement un courant émetteur pur, fourni par un circuit électrique oscillant haute fréquence de puissance.

Pour la mise en oeuvre du même procédé, WO 96/03928 décrit une sonde flexible susceptible de se conformer à la partie du corps contre laquelle elle est appliquée, comportant des parties non-conductrices inférieure/interne et supérieure/externe. Au moins la partie inférieure/interne est dotée de cavités présentant une pluralité de points conducteurs d'émission électromagnétique distincts formés par des parties de spires d'une bobine disposés en retrait par rapport à la surface inférieure/interne de la sonde.

Dans une autre évolution de la technique, le brevet français 2 589 067 décrit un procédé et un dispositif basés sur la constatation qu'un champ électrique appliqué à la peau en combinaison avec un rayonnement électromagnétique laser permet de modifier les propriétés d'absorption de ladite peau.

La demande de brevet FR 2 680 965 constitue l'état de la technique le plus proche. Ses caractéristiques forment le préambule de la revendication 1.

### EXPOSE DE L'INVENTION

La présente invention a pour objet une sonde pour l'application d'un flux d'énergie électromagnétique haute fréquence sur la peau (utilisable notamment dans les procédés décrits dans WO 93/04636 et le brevet français 2 589 067, ou autre procédé nécessitant l'application d'un gel conducteur), cette sonde présentant une surface d'appui non-conductrice destinée à être appliquée sur la peau et présentant dans son épaisseur au moins une cavité débouchant par un orifice dans la surface d'appui ainsi qu'au moins un élément inducteur de champ électromagnétique destiné à émettre un champ électromagnétique à travers ladite cavité et son orifice.

La sonde selon l'invention est caractérisée en ce que ledit élément inducteur se trouve dans son épaisseur à un niveau intermédiaire de la profondeur de ladite cavité, cet élément inducteur s'étendant au moins partiellement autour et/ou au voisinage de la paroi de ladite cavité.

D'autres caractéristiques de la sonde selon l'invention sont exposés dans les revendications 2-15. L'invention concerne également l'utilisation de cette sonde pour des traitements cosmétiques de la peau, comme exposé dans les revendications 16-18, et un procédé de traitement cosmétique de la peau selon les revendications 19-23.

Lors de l'utilisation de la sonde, on applique par exemple un mélange de gel/produit traitant sur la surface de la peau à traiter et/ou dans les cavités de la surface d'appui de la sonde de manière que ce mélange serve d'interface conducteur entre la surface d'appui de la sonde flexible et la peau.

La sonde selon l'invention présente divers avantages. Par exemple, la disposition intermédiaire de l'élément inducteur de champ magnétique permet d'agrandir la cavité et/ou de la rendre traversante dans l'épaisseur de la sonde, de manière à pouvoir installer une source d'émission électromagnétique laser dans l'épaisseur ou sur la face supérieure/externe de la sonde. Cet arrangement permet l'application simultanée aisée d'un champ électromagnétique et d'un rayonnement électromagnétique laser sur la peau, comme décrit dans le brevet français 2 589 067. En outre, la disposition intermédiaire de l'élément inducteur de champ magnétique permet une fabrication aisée des sondes par perforation. Par ailleurs, la disposition des éléments inducteurs de champ magnétique au moins partiellement autour et/ou au voisinage de la paroi des cavités permet de les disposer en couches successives, ce qui permet de multiplier le nombre de points d'émission de champ électromagnétique dans chaque cavité. Cette disposition permet en outre d'augmenter la quantité de mélange gel/produit traitant dans les cavités soumis au champ électromagnétique produit par les multiples points d'émission. Ceci augmente l'intensité du champ électromagnétique appliqué au mélange gel/produit et à la peau, et ainsi le pouvoir traitant des produits appliqués.

Les éléments inducteurs de champ électromagnétique sont par exemple constitués par des spires aplaties, ou par un réseau ou une grille constituée de matériau conducteur. Le matériau conducteur est de préférence une feuille conductrice en résine de silicone, un tissu de fibres conductrices telles des fibres de carbone, ou un textile non tissé dont les fibres sont conductrices.

Dans les formes d'exécution comprenant une source de rayonnement électromagnétique laser, celle-ci peut être constituée soit par une diode d'émission laser logée dans l'épaisseur ou sur la face supérieure/externe de la sonde, soit par une fibre optique reliée à une source laser externe à la sonde. Dans ces mêmes formes d'exécution, un arrangement possible est de disposer les cavités pratiquées dans la surface d'appui en réseau ou en lignes formant des figures géométriques diverses, et de disposer les sources laser de manière régulière dans ledit réseau ou lesdites lignes.

Si désiré, l'application du flux d'énergie électromagnétique peut suivre un programme déterminé par les moyens générateurs d'impulsions électriques et les moyens d'excitation de sources laser, par exemple en alternant les deux types de rayonnement électromagnétique et/ou en en variant l'intensité.

La sonde selon l'invention peut être utilisée, en combinaison avec un gel traitant, pour le traitement cosmétique de la peau, notamment l'épilation longue durée, la calvitie, la couperose et les varicosités veineuses et capillaires.

### BREVE DESCRIPTION DES DESSINS

D'autres caractéristiques de l'invention ressortiront à la lecture de la description qui suit, donnée à titre d'exemple, en se référant aux dessins schématiques annexés dans lesquels :
la Figure 1 est une vue en perspective d'une première forme d'exécution de la sonde selon l'invention, constituée d'un corps maniable allongé avec source laser;
la Figure 2 est une vue partielle en coupe axiale selon la ligne II-II de la sonde représentée à la Figure 1;
la Figure 3 est une vue de dessous d'une feuille faisant partie d'une deuxième forme d'exécution de la sonde selon l'invention;
la Figure 4 est une vue en coupe d'une sonde comportant la feuille de la Figure 3, selon la ligne IV-IV de la Figure 3;
la Figure 5 est une vue de dessous d'une troisième forme d'exécution de la sonde selon l'invention; et
la Figure 6 est une vue en coupe selon la ligne VI-VI de la sonde représentée à la Figure 5.

### DESCRIPTION DETAILLEE

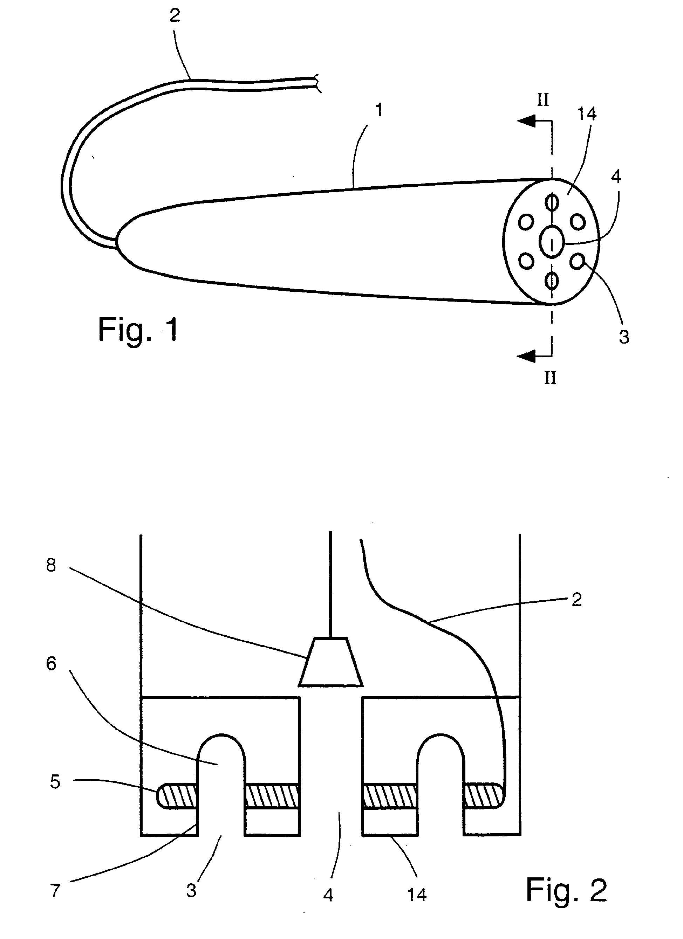

La sonde représentée aux Figures 1 et 2 comporte un organe maniable de contact 1 de forme allongée, relié, à l'une de ses extrémités, à une amenée de courant 2 et qui présente à son autre extrémité une surface d'appui 14 circulaire. L'amenée de courant 2 alimente un élément inducteur de champ électromagnétique 5 disposé en retrait de la surface de contact, émettant à travers des cavités 6, au nombre de six dans cet exemple, débouchant dans la surface d'appui 14. L'élément inducteur de champ électromagnétique 5 est situé à un niveau intermédiaire de la profondeur des cavités 6, cet élément inducteur s'étendant au moins partiellement autour et/ou au voisinage de la paroi 7 des cavités 6. Une source de rayonnement électromagnétique laser 8 émet à travers une cavité additionnelle 4 située dans l'axe de l'organe 1, débouchant dans la surface d'appui 14 au centre des cavités 6. Ces cavités 6 ont typiquement un diamètre entre 3mm et 6mm tandis que la cavité 4 d'émission de rayonnement laser a typiquement un diamètre entre 6mm et 10mm.

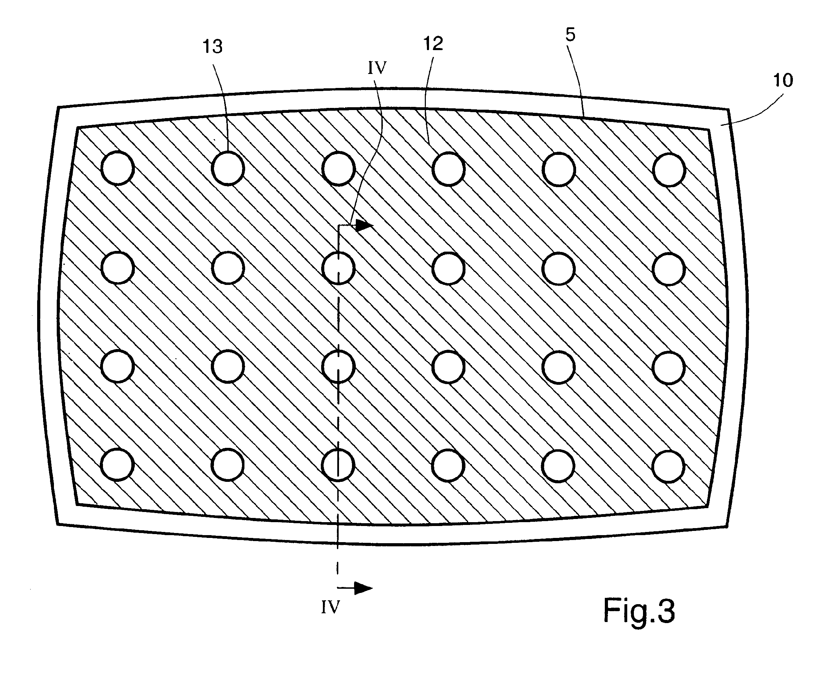

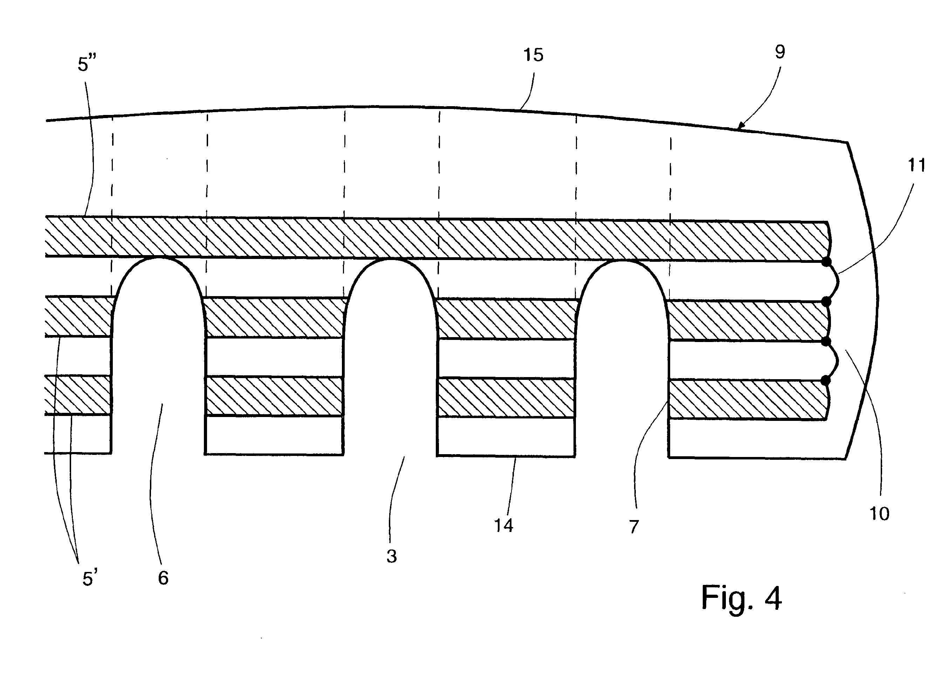

La forme d'exécution représentée aux Figures 3 et 4 est une sonde flexible 9 comprenant plusieurs (en l'espèce, quatre) couches de caoutchouc flexible entre lesquelles sont intercalées plusieurs (en l'espèce trois) éléments inducteurs 5 occupant la quasi totalité de la sonde flexible, excepté son pourtour non-conducteur 10. Les éléments inducteurs 5 sont disposés en plusieurs couches connectées électriquement entre elles par des connections 11, et reliées à une amenée de courant non-representée. On distingue sur la Figure 4 deux éléments inducteurs 5' situés à des niveaux intermédiaires de la profondeur des cavités 6, ainsi qu'un élément inducteur 5" optionnel situé au fond des cavités 6. Chaque élément inducteur 5 est par exemple constitué d'un tissu de fibres de carbone tissé 12 présentant des perforations 13. La sonde flexible 9 présente une face inférieure/interne constituant la surface d'appui 14, et une face supérieure/externe 15. Les cavités 6 sont situées dans l'épaisseur de la feuille en matériau flexible et débouchent dans la surface inférieure/interne 14 par des orifices 3. En variante, représentées en pointillé sur la Figure 4, les cavités 6 sont perforantes et traversantes, ce qui facilite la fabrication.

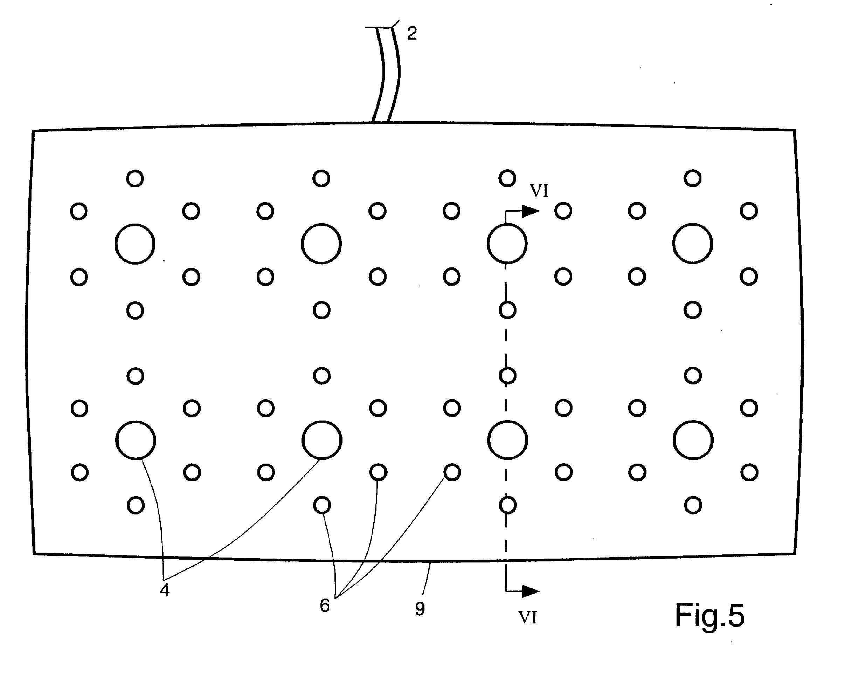

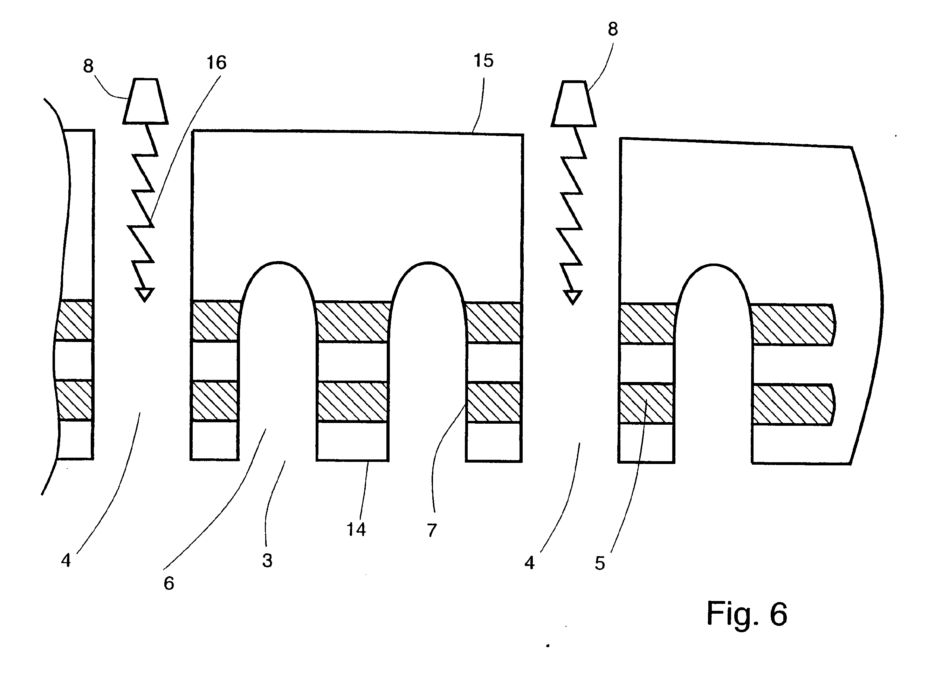

La forme d'exécution représentée aux Figures 5 et 6 est une sonde flexible 9 comportant des sources d'émission laser 8. Comme avant, la sonde se présente sous forme d' une feuille flexible pourvue d'une face inférieure/interne formant la surface d'appui 14, et une face supérieure/externe 15. La sonde comporte d'une part des cavités borgnes 6 disposées en réseau formant des figures géométriques, par exemple selon une disposition circulaire, et d'autre part des cavités traversantes additionnelles 4 disposées de manière régulière dans ledit réseau, permettant le passage du rayonnement électromagnétique laser 16 émis par la source 8. Les cavités 6 sont réparties autour des cavités traversantes 4 de manière à obtenir une répartition uniforme du rayonnement électromagnétique induit et du rayonnement laser. La sonde 9 est reliée par une amenée de courant 2 à des moyens générateurs d'impulsions électriques reliées aux éléments inducteurs 5, ainsi qu'à des moyens d'excitation des sources laser 8. Des exemples de ces deux types de moyens sont décrits par exemple dans le brevet français 2 589 067. En outre, avantageusement, la source laser comporte une lentille permettant la défocalisation du faisceau laser.

Si désiré, les différentes exécutions de la sonde 9 comportent en outre un interrupteur agencé de manière à activer l'application du flux d'énergie électromagnétique haute fréquence et l'excitation des sources laser lorsque la sonde est mise en contact avec la peau et/ou le gel. Cette caractéristique augmente la sécurité d'utilisation de la sonde, notamment dans les formes d'exécution comportant une ou plusieurs sources électromagnétiques laser. L'interrupteur, relié aux moyens générateurs d'impulsions électriques et aux moyens d'excitation de sources laser grâce à l'amenée de courant 2, peut être mécanique, optoélectronique, à impédance/capacitance de la peau et/ou du gel, ou de tout autre type connu de l'homme de l'art. Dans une variante, cet interrupteur est couplé avec un dispositif permettant de temporiser l'enclenchement ou le déclenchement de l'application du flux d'énergie électromagnétique lorsque la sonde est mise en contact avec la peau et/ou le gel, ou lorsqu'elle en est retirée.

Typiquement, les impulsions créant le champ électromagnétique sont des impulsions d'activation statique ayant des durées allant de 1 microseconde à 1 seconde, et ayant des fréquences de modulation à rapport constant de 5Hz à 1000Hz. Le rayonnement laser a normalement une énergie de 0.5 mW à 150 mW. Cependant, on peut utiliser des énergies plus élevées, de 500 mW et davantage, dans la mesure où le faisceau est défocalisé par une lentille afin de créer un champ de plus grande surface.

Le gel chargé utilisé pour le procédé de traitement est de préférence composé d'un gel conducteur non-polymérisable d'un type usuel pour le couplage ultrasonique d'électrodes sur la peau, en mélange avec un produit traitant. Le gel a un pH neutre et est, par exemple, à base gélifiante composée de trithanolaminepropylène glycolcarboxyvinylique. La composition du produit traitant dépend de l'action souhaitée. Pour une action dépilatoire, on peut choisir un produit à effet d'atrophie progressif de la racine du poil, par exemple une lotion post-épilatoire du type utilisé d'habitude immédiatement après une épilation à la cire, ainsi que les jours suivants. De telles lotions comportent des extraits de plantes, des huiles essentielles, de l'eau déminéralisée et éventuellement d'autres composants, par exemple des polyoxyéthylènes. Ces produits, connus parfois sous la dénomination "modérateur de la repousse des poils", sont sans danger d'utilisation toxique ou autre.

Pour un traitement de la calvitie, on peut mélanger le gel avec, par exemple, du minoxydil, ou tout autre produit favorisant la repousse des cheveux. Un mélange de 50:50 vol% de gel et de minoxydil a donné des résultats satisfaisants.

Afin de ne pas compromettre les propriétés conductrices du gel, la part du produit actif ou traitant n'excédera en général pas 50%, usuellement moins de 25%, en poids du gel (% en poids = % en volume). On peut ajouter de l'alcool, du chlorure de sodium et/ou d'autres substances pour améliorer la conductivité du produit, et/ou comme agents conservateurs.

Des tests ont démontré que l'application d'une énergie à haute fréquence sur le produit traitant seul ou sur le gel seul n'aboutit à aucun effet spécial, tandis qu'en mélange, on obtient une bonne pénétration du produit traitant entraîné par la solution conductrice provenant du gel. Il semble que la conduction de l'énergie électromagnétique et électrique suit le chemin de moindre résistance offert par le mélange du gel et de la lotion conductrice appliqué à la surface de la peau, et ensuite autour de la racine du poil ou du cheveu par la seule lotion conductrice qui pénètre dans le follicule pileux et la tige du poil. Le gel permet un dégagement progressif de la lotion active et sa pénétration, sous l'action conjuguée des champs électromagnétique et électrique. Dans les formes d'exécution comportant des couches successives d'éléments inducteurs, la multiplication des points d'émission de champ électromagnétique et la quantité plus importante de mélange gel/produit traitant présent dans les cavités permet une pénétration plus complète du produit traitant. Cet effet est en outre renforcé par l'adjonction d'un rayonnement électromagnétique laser.

Comme illustré aux Figures 1 et 6, les éléments inducteurs de champ électromagnétique 5 entourent également les cavités traversantes 4, et forment une partie des parois de ces cavités. Ainsi, le gel dans ces cavités 4 est à la fois ionisée par l'action du champ électromagnétique et traversée par le rayon laser. Cette action simultanée produit un effet synergique, et favorise une longue durée d'action sans danger sur la peau.

Avantageusement, la sonde selon l'invention fait partie d'un set comportant: une ou plusieurs sondes rigides selon l'invention adaptées pour le traitement de différentes parties du corps, chaque sonde rigide comportant une source laser (voir la Figure 1) ou plusieurs sources laser disposées dans un organe rigide maniable, par exemple trois sources dans des cavités 4 disposées symmétriquement autour de l'axe de l'organe; plusieurs sondes flexibles de dimensions différentes, avec source laser (par exemple selon les Figures 5 et 6) ou sans source laser (par exemple, selon les Figures 3 et 4, ou tel que décrit dans WO 96/03928); et au moins un embout porte aiguille pour la microthermolyse ou l'électrocoagulation. Toutes ces sondes et embouts sont interchangeables et peuvent être commandés par une unité centrale, permettant une très grande variété de traitements.

## Revendications

1. Sonde pour l'application d'un flux d'énergie électromagnétique haute fréquence sur la peau, la sonde présentant une surface d'appui non-conductrice (14) destinée à être appliquée sur la peau et présentant dans son épaisseur au moins une cavité (6) débouchant par un orifice (3) dans la surface d'appui ainsi qu'au moins un élément inducteur de champ électromagnétique (5,5') destiné à émettre un champ électromagnétique à travers ladite cavité et son orifice (3),
**caractérisée en ce que** ledit élément inducteur (5,5') se trouve dans ladite épaisseur à un niveau intermédiaire de la profondeur de ladite cavité (6), cet élément inducteur s'étendant au moins partiellement autour et/ou au voisinage de la paroi de ladite cavité.

2. Sonde selon la revendication 1, **caractérisée en ce que** le ou les éléments inducteurs (5,5') sont constitués de spires de forme aplatie.

3. Sonde selon la revendication 1, **caractérisée en ce que** le ou les éléments inducteurs sont constitués d'une feuille de matériau conducteur présentant des perforations (13) ou découpes, formant partie de ladite cavité (6).

4. Sonde selon la revendication 1, **caractérisée en ce que** le ou les éléments inducteurs (5,5') sont constitués d'une trame tissée, comportant des fibres conductrices.

5. Sonde selon la revendication 1, **caractérisée en ce que** le ou les éléments inducteurs (5,5') sont constitués d'un textile non tissé comportant des fibres conductrices.

6. Sonde selon la revendication 1, **caractérisée en ce que** le ou les éléments inducteurs (5,5') sont constitués d'une trame tissée en fibre de carbone.

7. Sonde selon la revendication 1, **caractérisée en ce que** au moins deux éléments inducteurs (5') sont disposés en couches distinctes espacées l'une de l'autre ou les unes des autres dans l'épaisseur de la sonde.

8. Sonde selon la revendication 7, comportant au moins une cavité borgne (6) débouchant dans la surface d'appui (14), **caractérisée en ce qu'**elle comporte en outre un élément inducteur additionnel (5") situé au fond de la ou des cavités borgnes (6).

9. Sonde selon l'une quelconque des revendications 1 à 8, **caractérisée en ce qu'**elle comporte au moins une source de rayonnement électromagnétique laser (8) disposée dans ou dans l'alignement d'au moins une cavité (4) débouchant dans la surface d'appui, de manière à agir de concert avec le rayonnement électromagnétique émis par ledit élément inducteur (5,5').

10. Sonde selon la revendication 9, **caractérisé en ce que** la source laser comporte au moins une diode d'émission laser intégrée à la sonde ou au moins une fibre optique reliée à au moins un dispositif d'émission laser externe à la sonde.

11. Sonde selon l'une quelconque des revendications 1 à 10, **caractérisée en ce qu'**elle se présente sous forme d'un organe maniable de contact avec la peau (1), cet organe étant constitué d'un corps allongé dont l'une des extrémités forme la surface d'appui (14).

12. Sonde selon l'une quelconque des revendications 1 à 10, **caractérisée en ce qu'**elle se présente sous forme d'une feuille flexible susceptible de se conformer à la partie du corps contre laquelle elle est appliquée, cette feuille flexible comportant une face inférieure/interne (14) et une face supérieure/externe (15) non-conductrices, au moins ladite surface inférieure comportant ladite surface d'appui (14).

13. Sonde selon la revendication 9, 10 ou l'une quelconque des revendications 11 à 12 lorsqu'elles dépendent de la revendication 9, **caractérisée en ce que** des cavités (6) débouchant dans la surface d'appui sont disposées en réseau ou en alignements formant des figures géométriques diverses, et **en ce que** des sources laser (8) sont disposées de manière régulière dans ledit réseau ou lesdits alignements.

14. Sonde selon la revendication 7 ou l'une quelconque des revendications 8 à 13 lorsqu'elles dépendent de la revendication 7, **caractérisée en ce qu'**elle est formée de plusieurs couches distinctes empilées en sandwich, comportant des cavités traversantes (4,6) produites par perforation.

15. Sonde selon l'une quelconque des revendications précédentes, **caractérisée en ce qu'**elle comporte en outre un interrupteur agencé de manière à activer l'application du flux d'énergie électromagnétique haute fréquence, et éventuellement la source laser, lorsque la sonde est mise en contact avec la peau et/ou le gel.

## Claims

1. A probe for applying a flux of high-frequency electromagnetic energy to the skin, the probe being provided with a non-conductive bearing surface (14) adapted to be applied to the skin, this surface having in its thickness at least one cavity (6) that opens out via an orifice (3) into the bearing surface as well as at least one inductor element (5,5') for inducing an electromagnetic field and arranged to emit an electromagnetic field through said cavity and its orifice (3).
**characterized in that** said inductor element (5,5') is located in said thickness at an intermediate level of the depth of the cavity (6), this inductor element extending at least partly around and/or being in the proximity of said cavity's wall.

2. A probe according to claim 1, **characterized in that** the inductor element(s) (5, 5') is/are constituted of flattened windings.

3. A probe according to claim 1, **characterized in that** the inductor element(s) is/are constituted of a sheet of conductive material having perforations (13) or cutouts forming a part of said cavity (6).

4. A probe according to claim 1, **characterized in that** the inductor element(s) (5, 5') is/are constituted of a non-woven fabric including conductive fibers.

5. A probe according to claim 1, **characterized in that** the inductor element(s) (5, 5') is/are constituted of a non-woven fabric including conductive fibers.

6. A probe according to claim 1, **characterized in that** the inductor element(s) (5, 5') is/are constituted of a woven fabric of carbon fibers.

7. A probe according to claim 1, **characterized in that** at least two inductor element(s) (5') are arranged in discrete layers spaced apart from one another in the thickness of the probe.

8. A probe according to claim 7, having at least one blind cavity (6) leading into the bearing surface (14), **characterized in that** it further comprises an additional inductor element (5") situated at the bottom of the blind cavity/cavities (6).

9. A probe according to any one of claims 1 to 8, **characterized in that** it comprises at least one source (8) of laser electromagnetic radiation arranged in or in alignment with at least one opening (4) leading into the support surface, in such a manner as to act together with the electromagnetic radiation emitted by said inductor element(s) (5,5').

10. A probe according to claim 9, **characterized in that** the laser source comprises at least one laser-emitting diode integrated in the probe, or at least one optical fiber connected to at least one laser-emitting device external to the probe.

11. A probe according to any one of claims 1 to 10, **characterized in that** it is in the form of a handleable member (1) for contacting the skin, this member being formed of an elongate body one end of which forms the bearing surface (14).

12. A probe according to any one of claims 1 to 10, **characterized in that** it is in the form of a flexible sheet able to conform to a part of the body against which it is applied, this flexible sheet having a lower/inner face (14), and a non-conductive upper/external face (15), at least said lower face including said support surface (14).

13. A probe according to claim 9 or 10, or claim 11 or 12 when depending on claim 9, **characterized in that** the cavities (6) leading into the support surface are arranged in a repeating pattern or in alignments forming various geometrical figures, and **in that** the laser sources (8) are arranged regularly relative to said pattern or lines.

14. A probe according to claim 7, or any one of claims 8 to 13 when depending on claim 7, **characterized in that** it is formed of several discrete layers piled as a sandwich and including through-holes (4,6) produced by perforation.

15. A probe according to any preceding claim, **characterized in that** it further includes a switch arranged so as to activate the flux of high-frequency electromagnetic energy, and possibly also excitation of the laser source, when the probe comes into contact with the skin and/or with the gel.

## Patentansprüche

1. Sonde zum Applizieren eines hochfrequenten elektromagnetischen Energieflusses auf die Haut, wobei die Sonde eine nichtleitende Auflagefläche (14) aufweist, die zum Applizieren auf die Haut bestimmt ist und die in ihrer Dickenrichtung wenigstens einen Hohlraum (6) aufweist, der über eine Öffnung (3) in die Auflagefläche mündet, sowie über wenigstens ein Induktionselement (5, 5') für das elektromagnetische Feld verfügt, das dazu eingerichtet ist, ein elektromagnetisches Feld durch den Hohlraum und dessen Öffnung (3) hindurch abzugeben, **dadurch gekennzeichnet, dass** sich das Induktionselement (5, 5') in der Dickenrichtung im mittleren Bereich der Tiefe des Hohlraumes (6) befindet, wobei sich das Induktionselement wenigstens teilweise um die und/oder in der Nähe der Wand des Hohlraumes erstreckt.

2. Sonde nach Anspruch 1, **dadurch gekennzeichnet, dass** das oder jedes Induktionselement (5, 5') aus Windungen mit einer abgeflachten Gestalt gebildet ist.

3. Sonde nach Anspruch 1, **dadurch gekennzeichnet, dass** das oder jedes Induktionselement aus einer Lage aus leitendem Material gebildet ist, das Perforationen (13) oder Ausstanzungen aufweist, die einen Teil des Hohlraumes (6) bilden.

4. Sonde nach Anspruch 1, **dadurch gekennzeichnet, dass** das oder jedes Induktionselement (5, 5') aus einem gewebten Textilstück gebildet ist, das leitende Fasern aufweist.

5. Sonde nach Anspruch 1, **dadurch gekennzeichnet, dass** das oder jedes Induktionselement (5, 5') aus einem nicht gewebten Vlies gebildet ist, das leitende Fasern aufweist.

6. Sonde nach Anspruch 1, **dadurch gekennzeichnet, dass** das oder jedes Induktionselement (5, 5') aus einem gewebten Textilstück aus Kohlefasern gebildet ist.

7. Sonde nach Anspruch 1, **dadurch gekennzeichnet, dass** wenigstens zwei Induktionselemente (5') in unterschiedlichen Lagen angeordnet sind, die jeweils in der Dickenrichtung der Sonde voneinander beabstandet sind.

8. Sonde nach Anspruch 7, die wenigstens einen geschlossenen Hohlraum (6) aufweist, der in der Auflagefläche (14) mündet, **dadurch gekennzeichnet, dass** sie weiterhin ein zusätzliches Induktionselement (5") aufweist, das im Bereich des Grundes des oder jedes geschlossenen Hohlraums (6) angeordnet ist.

9. Sonde nach einem der Ansprüche 1 bis 8, **dadurch gekennzeichnet, dass** sie wenigstens eine Laserstrahlungsquelle (8) aufweist, die in oder fluchtend mit wenigstens einem Hohlraum (4) angeordnet ist, der in die Auflagefläche mündet, so dass sie zusammen mit der von dem Induktionselement (5, 5') emittierten elektromagnetischen Strahlung wirkt.

10. Sonde nach Anspruch 9, **dadurch gekennzeichnet, dass** die Laserquelle wenigstens eine Laserdiode aufweist, die in die Sonde integriert ist, oder über wenigstens eine optische Faser verfügt, die mit wenigstens einer Laserstrahlungsanordnung außerhalb der Sonde verbunden ist.

11. Sonde nach einem der Ansprüche 1 bis 10, **dadurch gekennzeichnet, dass** sie in Gestalt eines Handstückes zum Kontakt mit der Haut (1) ausgebildet ist, wobei das Handstück durch einen länglichen Körper gebildet ist, bei dem ein Ende die Auflagefläche (14) bildet.

12. Sonde nach einem der Ansprüche 1 bis 10, **dadurch gekennzeichnet, dass** sie in Gestalt einer flexiblen Lage aufgebaut ist, die dazu eingerichtet ist, sich an den Teil des Körpers anzupassen, auf den sie appliziert wird, wobei die flexible Lage eine untere/innenseitige Seite (14) und eine obere/äußere Seite (15) aufweist, die nicht leitend sind, wobei wenigstens die untere Seite die Auflagefläche (14) aufweist.

13. Sonde nach Anspruch 9, 10 oder einem der Ansprüche 11 bis 12 soweit auf Anspruch 9 rückbezogen, **dadurch gekennzeichnet, dass** die in die Auflagefläche mündenden Hohlräume (6) gitternetzartig oder in Gestalt von verschiedenen geometrischen Anordnungen ausgerichtet angeordnet sind und dass die Laserquellen (8) regelmäßig innerhalb des Netzes oder der ausgerichteten Anordnungen angeordnet sind.

14. Sonde nach Anspruch 7 oder einem der Ansprüche 8 bis 13 soweit auf Anspruch 7 rückbezogen, **dadurch gekennzeichnet, dass** sie mit mehreren unterschiedlichen, sandwichartig aufeinandergestapelten Lagen gebildet ist, die die durch Perforation ausgebildeten, sich erstreckenden Hohlräume (4, 6) aufweisen.

15. Sonde nach einem der vorangehenden Ansprüche, **dadurch gekennzeichnet, dass** sie weiterhin einen Unterbrecher aufweist, der zum Aktivieren der Applikation des hochfrequenten elektromagnetischen Energieflusses eingerichtet ist, und weiterhin gegebenenfalls über eine Laserquelle verfügt, während die Sonde in Kontakt mit der Haut und/oder dem Gel gebracht wird.
